# EUROPEAN PATENT APPLICATION

(11) **EP 2 845 625 A1**
(43) Date of publication of application: **11.03.2015**
(21) Application number: 13182985.5
(22) Date of filing: 04.09.2013
(51) Int. Cl.: A61P 25/04, A61K 9/20, A61K 31/137

(54) **Tapentadol for use in the treatment of fibromyalgia and chronic fatigue syndrome**

(71) Applicant: Grünenthal GmbH, 52078 Aachen (DE)
(72) Inventor: Schiene, Klaus, 41363 Jüchen (DE); Bloms-Funke, Petra, 52146 Würselen (DE)
(74) Representative: Brosch, Oliver

(57) **Abstract**

The invention relates to tapentadol for use in the treatment of fibromyalgia or chronic fatigue syndrome.

## Description

The invention relates to tapentadol for use in the treatment of fibromyalgia and chronic fatigue syndrome. The treatment is effective for treating fibromyalgia-related pain and fatigue.

Fibromyalgia syndrome (FMS) is a chronic, widespread musculoskeletal pain and fatigue disorder, estimated to affect 2-4% of the population. FMS is characterized by a generalized heightened perception of sensory stimuli. Patients with FMS display abnormalities in pain perception in the form of both allodynia (pain with innocuous stimulation) and hyperalgesia (increased sensitivity to painful stimuli). The syndrome, as defined by the American College of Rheumatology's criteria, involves the presence of pain for over 3 months duration in all four quadrants of the body, as well as along the spine. In addition, pain is elicited at 11 out of 18 "tender points" upon palpation. In addition to muscle pain and fatigue, many patients commonly develop sleep and mood disorders (e.g., anxiety, depression). Patients also show a higher incidence of stress-related symptoms.

Chronic fatigue syndrome (CFS) is a debilitating disorder characterized by profound tiredness or fatigue. Patients with CFS may become exhausted with only light physical exertion, and must often function at a level of activity substantially lower than their capacity before the onset of illness. In addition to the key defining characteristic of fatigue, CFS patients generally report various nonspecific symptoms, including weakness, muscle aches and pains, excessive sleep, malaise, fever, sore throat, tender lymph nodes, impaired memory and/or mental concentration, insomnia, and depression. Like patients with FMS, patients with CFS suffer from disordered sleep, localized tenderness, and complaints of diffuse pain and fatigue.

Owing to their common symptomology, FMS and CFS are thought to be related. However, they manifest different major symptoms. Whereas pain is the major symptom reported by patients with FMS, fatigue is the major symptom reported by patients with CFS.

It is an object of the invention to provide a medicament containing at least one pharmacologically active compound that is useful in the treatment of fibromyalgia and chronic fatigue syndrome and that preferably has advantages over the medicaments known in prior art for the treatment of FMS and CMS.

This object has been achieved by the subject-matter of the patent claims and the specification.

The invention therefore relates to the use of tapentadol, preferably contained in a medicament, for use in the treatment of fibromyalgia or chronic fatigue syndrome.

Tapentadol, i.e. (1R,2R)-3-(3-dimethylamino-1-ethyl-2-methyl-propyl)-phenol (CAS no. 175591-23-8), is a synthetic, centrally acting analgesic which is effective in the treatment of moderate to severe, acute or chronic pain. The compound can be used in the form of its free base or as a salt or solvate. The production of the free base is known, for example, from EP-A 693 475. For the purposes of the present invention tapentadol includes (1 R,2R)-3-(3-dimethylamino-1-ethyl-2-methyl-propyl)-phenol as well as physiologically acceptable salts and solvates thereof, in particular (1 R,2R)-3-(3-dimethylamino-1-ethyl-2-methyl-propyl)-phenol hydrochloride.

The invention further relates to a medicament containing tapentadol for use in the treatment of fibromyalgia or chronic fatigue syndrome. The medicament is preferably in a solid medicinal form. Semisolid, liquid or pasty medicinal forms are also possible.

In an embodiment, the invention relates to said medicament and its use in the treatment of fibromyalgia or chronic fatigue syndrome wherein tapentadol is formulated in an controlled release form. Controlled release forms of tapentadol are known. Such medicaments may be particularly useful for treating chronic conditions. The controlled release of tapentadol can, for example, be achieved by retardation by means of a matrix, a coating or release systems with an osmotic action cf. the respective formulaions in US 2005/0058706A which content is hereby incorporated by reference.

Controlled release of tapentadol is possible from formulations such as those for oral, rectal or percutaneous administration. Preferably, the medicament is formulated for once-daily administration, for twice-daily administration (bid) or for thrice-daily administration, with twice-daily administration (bid) being particularly preferred.

In another embodiment, the invention relates to said medicament and its use in the treatment of fibromyalgia or chronic fatigue syndrome wherein tapentadol is formulated in an immediate release form. Immediate release forms of tapentadol are known and are commercially available under the tradename Palexia and Nucynta.

In another embodiment, the invention relates to said medicament and its use in the treatment of fibromyalgia or chronic fatigue syndrome which is formulated for oral administration. Such formulations may contain suitable excipients and additives such as, e.g. disintegrants, lubricants, binders, fillers, mould release agents, optionally solvents, flavourings, sugar, in particular carriers, diluents, colorants, antioxidants, etc. Oral solutions are known from US 2013/0022670A which content is hereby incorporated by reference.

Suitable for oral administration are medicaments and/ or preparations which are in the form of tablets, chewable tablets, dragees, capsules, granules, drops, juices or syrups; suitable for parenteral, topical and inhalative administration are solutions, suspensions, easily reconstituted dry preparations and sprays. A further possibility is suppositories for use in the rectum. Use in a depot in dissolved form, a carrier foil or a plaster, optionally with the addition of means to encourage penetration of the skin, are examples of suitable percutaneous administration forms.

According to the invention, the medicament containing tapentadol can also present in pharmaceutical forms that are adapted for other than oral administration routes (cf. US 2012/0225950A, and US 2012/0225951A which contents are hereby incorporated by reference).

Depending upon the formulation, such pharmaceutical forms may contain suitable additives and/or excipients. Suitable additives and/or excipients are all substances for achieving generally known galenic formulations. The selection of these excipients and the amounts to be used depend upon the anticipated administration route (e.g. orally, intravenously, intraperitoneally, intradermally, intramusuclarly, intranasally, buccally or topically.administration routes, or buccal, sublingual, transmucosal, rectal, intralumbar, intraperitoneal, transdermal, intravenous, intramuscular, intragluteal, intracutaneous and subcutaneous administration routes).

For suppositories, it is possible to use inter alia waxes or fatty acid esters and for parenteral means of application, carriers, preservatives, suspension aids, etc.

Suitable physiologically acceptable salts of tapentadol which can be used according to the invention include salts of inorganic acids, such as e.g. hydrogen chloride, hydrogen bromide and sulfuric acid, and salts of organic acids, such as methanesulfonic acid, fumaric acid, maleic acid, acetic acid, oxalic acid, succinic acid, malic acid, tartaric acid, mandelic acid, lactic acid, citric acid, glutaminic acid, acetylsalicylic acid, nicotinic acid, aminobenzoic acid, a-lipoic acid, hippuric acid and aspartic acid. Tapentadol can also be present as any mixture of the salts of the above-mentioned organic and inorganic acids. The most preferred salt to be used according to the invention is tapentadol hydrochloride, which can be used in an amorphous or polymorphic form (cf. US2007/213405 A which content is incorporated by reference).

The medicinal product, the medicament or the pharmaceutical composition according to the present invention contains tapentadol in an amount of 0.001 to 99.999 % by weight, preferably 0.1 to 99.9 % by weight, more preferably 1.0 to 99.0 % by weight, even more preferably 2.5 to 80 % by weight, most preferably 5.0 to 50 % by weight and in particular 7.5 to 40 % by weight, based on the total weight of the medicament and preferably is formulated as tablets, capsules, pellets or granules.

The medicinal product, the medicament or the pharmaceutical composition according to the present invention has a total mass in the range of from 25 to 2,000 mg, preferably 50 to 1,800 mg, more preferably 60 to 1,600 mg, even more preferably 70 to 1,400 mg, most preferably 80 to 1,200 mg and in particular 100 to 1,000 mg, and preferably is formulated as tablets, capsules, pellets or granules.

In a preferred embodiment of the invention the medicament contains Tapentadol having a controlled release from a matrix, and which medicament is in a solid and/or pressed and/or film-coated medicinal form and is formulated for oral administration, which medicament contains tapentadol in an amount of 0.001 to 99.999 % by weight, preferably 0.1 to 99.9 % by weight, more preferably 1.0 to 99.0 % by weight, even more preferably 2.5 to 80 % by weight, most preferably 5.0 to 50 % by weight and in particular 7.5 to 40 % by weight, based on the total weight of the medicament, and preferably is formulated as tablets, capsules, pellets or granules.

In another preferred embodiment of the invention the medicament contains tapentadol having a controlled release from a matrix, and which medicament is in a solid and/or pressed and/or film-coated medicinal form and is formulated for oral administration, which medicament contains tapentadol in an amount of 0.001 to 99.999 % by weight, preferably 0.1 to 99.9 % by weight, more preferably 1.0 to 99.0 % by weight, even more preferably 2.5 to 80 % by weight, most preferably 5.0 to 50 % by weight and in particular 7.5 to 40 % by weight, based on the total weight of the medicament, and which medicament has a total mass in the range of from 25 to 2,000 mg, preferably 50 to 1,800 mg, more preferably 60 to 1,600 mg, even more preferably 70 to 1,400 mg, most preferably 80 to 1,200 mg and in particular 100 to 1,000 mg and preferably is formulated as tablets, capsules, pellets or granules.

For the purpose of the specification, fibromyalgia or chronic fatigue syndrome shall include conditions and symptoms that are associated with fibromyalgia or chronic fatigue syndrome, particularly pain due to fibromyalgia and pain due to chronic fatigue syndrome.

When tapentadol is used for the treatment of fibromyalgia or of a condition associated with fibromyalgia, the fibromyalgia is preferably selected from fibromyositis, fibrositis, myofibrositis, diffuse myofascial pain syndrome, primary fibromyalgia, secondary fibromyalgia, fibromyalgia-fibromyositis syndrome, fibromyositis-fibromyalgia syndrome, and muscular rheumatism.

When tapentadol is used for the treatment of chronic fatigue syndrome or of a condition associated with chronic fatigue syndrome, the chronic fatigue syndrome is preferably selected from chronic fatigue and immune dysfunction syndrome, chronic fatigue disorder, chronic fatigue-fibromyalgia syndrome, myalgic encephalomyelitis, postviral fatigue syndrome, chronic infectious mononucleosis-like syndrome, and royal free disease.

The term controlled release as used herein refers to any type of release other than immediate release such as delayed release, prolonged release, sustained release, slow release, extended release and the like. These terms are well known to any person skilled in the art as are the means, devices, methods and processes for obtaining such type of release.

Besides the excipients mentioned before which can be used in the pharmaceutical forms or the medicament according to the invention further excipients are for example water, ethanol, 2-propanol, glycerin, ethylene glycol, propylene glycol, polyethylene glycol, polypropylene glycol, glucose, fructose, lactose, sucrose, dextrose, molasses, starch, modified starch, gelatin, sorbitol, inositol, mannitol, microcrystalline cellulose, methyl cellulose, carboxymethylcellulose, cellulose acetate, shellac, cetyl alcohol, polyvinylpyrrolidone, paraffins, waxes, natural and synthetic rubbers, acacia gum, alginates, dextran, saturated and unsaturated fatty acids, stearic acid, magnesium stearate, zinc stearate, glyceryl stearate, sodium lauryl sulfate, edible oils, sesame oil, coconut oil, groundnut oil, soybean oil, lecithin, sodium lactate, polyoxyethylene and propylene fatty acid ester, sorbitan fatty acid esters, sorbic acid, benzoic acid, citric acid, ascorbic acid, tannic acid, sodium chloride, potassium chloride, magnesium chloride, calcium chloride, magnesium oxide, zinc oxide, silicon dioxide, titanium oxide, titanium dioxide, magnesium sulfate, zinc sulfate, calcium sulfate, potash, calcium phosphate, dicalcium phosphate, potassium bromide, potassium iodide, talc kaolin, pectin, crospovidone, agar and bentonite.

The preparation of the medicinal product, the medicament and the pharmaceutical composition according to the present invention can be performed with the aid of means, devices, methods and processes which are well known in the prior art of pharmaceutical formulation, such as those described for example in *"*Remington's Pharmaceutical Sciences", ed AR Gennaro, 17th edition, Mack Publishing Company, Easton, pa. (1985), in particular in Part 8, Chapters 76 to 93.

For example, for a solid formulation, such as a tablet, tapentadol can be granulated with a pharmaceutical carrier, e.g. conventional tablet ingredients, such as maize starch, lactose, sucrose, sorbitol, talc, magnesium stearate, dicalcium phosphate or physiologically acceptable rubbers, and pharmaceutical diluents, such as water, for example, to form a solid composition containing the active substance in a homogeneous distribution. Here, a homogeneous distribution should be understood as meaning that the active substance is distributed uniformly throughout the entire composition so that this can be easily divided into equally effective single dose forms, such as tablets, capsules, dragees. The solid composition is then divided into single dose forms. The tablets or pills can also be coated or compounded in some other way in order to produce a dosage form with delayed release. Suitable coating means are inter alia polymers acids and mixtures of polymeric acids with materials such as shellac, for example, cetyl alcohol and/or cellulose acetate.

The medicament according to the invention may contain one or more further pharmaceutically active ingredients besides tapentadol. Preferred active ingredients are selected from celecoxib, rofecoxib, etoricoxib, valdecoxib, parecoxib, etodolac, meloxicam, nimesulide acemetacin, paracetamol, acetylsalicylic acid, bufexamac, diclofenac, diclofenac-sodium, diflunisal, dipyrone (metamizol), metamizol-sodium, ethenzamide, etofenamate, flufenamic acid, flurbiprofen, ibuprofen, indomethacin, isoxicam, kebuzone, ketoprofen, ketorolac, lonazolac, lornoxicam, meclofenamic acid, mefenamic acid, mofebutazone, nabumetone, naproxen, (+)-naproxen, (-)-ibuprofen, (+)-ibuprofen, naproxen, (+)-naproxen, niflumic acid, oxaprozine, oxyphenbutazone, phenylbutazone, piroxicam, propyphenazone, salicylamide, sulindac, tenoxicam, tiaprofenic acid, SC560, sulphasalazine and tolmetin norvaline (AP5), D-norvaline (D-AP5), 4-(3-phosphono-propyl)-piperazine-2- carboxylic acid (CPP), D-(E)-4-(3-phosphonoprop-2-enyl)piperazine-2-carboxylic acid (D-CPPene), cis-4-(phosphonomethyl)-2-piperidine carboxylic acid (Selfotel, CGS 19755), SDZ-220581, PD-134705, LY-274614, WAY-126090, kynurenic acid, 7-chloro-kynurenic acid, 7-chloro-thiokynurenic acid, 5,7-dichloro-knynurenic acid, 4-chlorokynurenine, 3-hydroxy-kynurenine, L-689,560, L-701,324, licostinel (ACEA-1021), CGP-68,730A, MDL-105,519, gavestinel (GV-150,526), GV-196,771A, ZD-9,379, MRZ-2/576, (+)-HA-966, dextromethorphan, dextrophan, BIII-277CL, dextropropoxyphene, ketobemidone, dextromethadone, D-morphine, amantadine, memantine, MRZ-2/579, ifenprodil, eliprodil, PD-196,860, nitroprusside, D-cycloserine, 1-aminocyclopropane-carboxylic acid, dizocilpine (MK 801), phencyclidine (PCP), ketamine, (R,S)-ketamine, (R)-ketamine, (S)-ketamine, remacemide, FPL-12,495, AR-R-15,896, methadone, sulfazocine, AN19/AVex-144, AN2/AVex-73, Besonprodil, CGX-1007, EAB-318, Felbamate and NPS-1407), gabapentin and pregabalin.

The amounts of tapentadol to be administered to patients may vary depending upon the weight of the patient, the method of administration and the severity of the disease and/or pain. Tapentadol may be administered in amounts up to its maximum daily dosage, which is known to those skilled in the art. In a preferred embodiment, the medicament contains tapentadol in an amount of 10 to 300 mg, more preferably 20 to 290 mg, even more preferably 30 to 280 mg, most preferably 40 to 260 mg, as an equivalent dose based on the free base.

In a preferred embodiment, the mean serum concentration of tapentadol, following twice-daily administration of the medicament over a period of at least three days, more preferably at least four days and in particular at least five days, is on average at least 5.0 ng/ml, at least 10 ng/ml, at least 15 ng/ml or at least 20 ng/ml, more preferably at least 25 ng/ml or at least 30 ng/ml, even more preferably at least 35 ng/ml or at least 40 ng/ml, most preferably at least 45 ng/ml or at least 50 ng/ml and in particular at least 55 ng/ml or at least 60 ng/ml. This means that tapentadol is administered over a period of at least three days twice daily and then, preferably 2 h after the administration, the serum concentration is measured. The authoritative numerical value is then obtained as the mean value for all the patients investigated.

In a preferred embodiment, the mean serum concentration of tapentadol in at the most 50% of the patient population, which preferably comprises at least 100 patients, more preferably in at the most 40%, even more preferably in at the most 30%, most preferably in at the most 20% and in particular in at the most 10% of the patient population, following twice-daily administration over a period of at least three days, more preferably at least four days and in particular at least five days, is on average less than 5.0 ng/ml, preferably less than 7.5 ng/ml, even more preferably less than 10 ng/ml, most preferably less than 15 ng/ml and in particular less than 20 ng/ml.

In a preferred embodiment, the mean serum concentration of tapentadol in at the most 50% of the patient population, comprising preferably at least 100 patients, more preferably in at the most 40%, even more preferably in at the most 30%, most preferably in at the most 20% and in particular in at the most 10% of the patient population, following twice-daily administration over a period of at least three days, more preferably at least four days and in particular at least five days, is on average more than 300 ng/ml, more preferably more than 275 ng/ml, even more preferably more than 250 ng/ml, most preferably more than 225 ng/ml and in particular more than 200 ng/ml.

Preferably, the mean serum concentration of tapentadol in at least 50% or 55% of the patient population, which preferably comprises at least 100 patients, more preferably in at least 60% or 65%, even more preferably in at least 70% or 75%, most preferably in at least 80% or 85% and in particular in at least 90% or 95% of the patient population, following twice-daily administration over a period of at least three days, more preferably at least four days and in particular at least five days, is on average in the range of from 1.0 ng/ml to 500 ng/ml, more preferably in the range of from 2.0 ng/ml to 450 ng/ml, even more preferably in the range of from 3.0 ng/ml to 400 ng/ml, most preferably in the range of from 4.0 ng/ml to 350 ng/ml and in particular in the range of from 5.0 ng/ml to 300 ng/ml.

In a preferred embodiment, the percentage standard deviation (coefficient of variation) of the mean serum concentration of tapentadol, preferably in a patient population of 100 patients, following twice-daily administration of the medicament over a period of at least three days, more preferably at least four days and in particular at least five days, is at the most ± 90%, more preferably at the most ± 70%, even more preferably at the most ± 50%, at the most ± 45% or at the most ± 40%, most preferably at the most ± 35%, at the most ± 30% or at the most ± 25% and in particular at the most ± 20%, at the most ± 15% or at the most ± 10%.

Preferably, the serum concentrations are average values, produced from measurements on a patient population of preferably at least 10, more preferably at least 25, even more preferably at least 50, even more preferably at least 75, most preferably at least 100 and in particular at least 250 patients. A person skilled in the art knows how to determine the serum concentrations of tapentadol. In this context, reference is made, for example, to TM Tzschentke et al, Drugs of the Future, 2006, 31 (12), 1053.

The medicament according to the invention can be provided as a simple tablet and as a coated tablet (e.g. as a film-coated tablet or dragee). The tablets are usually round and biconvex, but oblong shapes are also possible. Granules, spheroids, pellets or microcapsules, which are used to fill sachets or capsules or pressed into disintegrating tablets, are also possible.

Medicaments containing at least 0.001 to 99.999 % tapentadol, in particular low, active doses, are preferred in order to avoid side effects. The medicament contains preferably 0.01 % by weight to 99.99 % by weight tapentadol, more preferably 0.1 to 90 % by weight, even more preferably 0.5 to 80 % by weight, most preferably 1.0 to 50 % by weight and in particular 5.0 to 20 % by weight. To avoid side effects, it may be advantageous at the start of the treatment to increase the amount of tapentadol to be administered gradually (titration) to allow the body to become accustomed to the active substance slowly. Such a titration method is known from US 2009/012180A which content is hereby incorporated by reference

Particularly preferably, the medicament has an oral pharmaceutical form, which is formulated for twice-daily administration and contains tapentadol in an amount of 20 to 260 mg as an equivalent dose based on the free base.

In another one of its embodiments, the present invention relates to tapentadol for use in a method for the treatment of fibromyalgia or chronic fatigue syndrome.

In yet another one of its embodiments, the present invention relates to the use of tapentadol for the preparation of a medicament for the treatment of fibromyalgia or chronic fatigue syndrome.

In yet another one of its embodiments, the present invention relates to a method for treating fibromyalgia or chronic fatigue syndrome in a patient, preferably in a mammal, more preferably in a human, which comprises administering an effective and physiologically acceptable amount of tapentadol as described herein to a patient.

When tapentadol is used is for the treatment of fibromyalgia or of a condition associated with fibromyalgia, the fibromyalgia is preferably selected from fibromyositis, fibrositis, myofibrositis, diffuse myofascial pain syndrome, primary fibromyalgia, secondary fibromyalgia, fibromyalgia-fibromyositis syndrome, fibromyositis-fibromyalgia syndrome, and muscular rheumatism.

When tapentadol is used for the treatment of chronic fatigue syndrome or of a condition associated with chronic fatigue syndrome, the chronic fatigue syndrome is preferably selected from chronic fatigue and immune dysfunction syndrome, chronic fatigue disorder, chronic fatigue-fibromyalgia syndrome, myalgic encephalomyelitis, postviral fatigue syndrome, chronic infectious mononucleosis-like syndrome, and royal free disease.

Even if the medicaments according to the invention exhibit few side effects only, it may be advantageous, for example, in order to avoid certain types of dependency to use morphine antagonists, in particular naloxone, naltrexone and/or levallorphan, in addition to tapentadol.

The present invention also relates to a kit comprising a medicament containing tapentadol (dosage forms) according to the invention.

### Pharmacological Methods:

The following models are suitable for testing a pharmaceutically active substance with regard to its activity in fibromyalgia.

### Model 1:

Patients suffering from fibromyalgia report an increased incidence of stress. It is well established that acute stress exposure can increase the discharge activity and norepinephrine release from noradrenergic *locus coeruleus* (LC) neurons. The LC contains the largest aggregate of noradrenergic neurons in the mammalian brain. Chronic exposure to stress can alter the response of LC neurons to subsequent stress exposure. It is to be expected that a compound which is able to inhibit the discharge activity within the LC would be appropriate for treating stress-related symptoms.

### Method:

The experiments can all be carried out in male albino Sprague-Dawley rats. Animals are maintained under standard laboratory conditions (for example: 21 °C, 12h light/dark cycle, lights on at 8:00 AM, food and water ad libitum). Every effort should be made to minimize animal suffering and to use the minimum possible number of animals. For example, animal use procedures can be conformed to European Ethical Standards (86/609-EEC) and Spanish law (RD1201/2005) for the care and use of laboratory animals. The experimental protocols can be reviewed and approved by the Committee for Animal Experimentation at the University of Cádiz and complied with the International Association for the Study of Pain Ethical Guidelines.

Rats are anesthetized with chloral hydrate (400 mg/kg i.p.); subsequently, a cannula is inserted into the trachea and the right jugular vein is cannulated for systemic i.v. injections of anaesthetic and drugs. Supplemental doses of anaesthetic are given to prevent any nociceptive reaction to pinching of the hind paw. Body temperature is maintained at 37 °C with a heating pad. The rat is placed in a stereotaxic frame with its head at a 15° angle to the horizontal plane (nose down). To approach the *locus coeruleus,* the skull is exposed, and a hole (approximately 3 mm diameter) is drilled for the insertion of the recording electrode at 1.1 mm lateral to the midline and 3.7 mm posterior to the lamboid fontanel over the cerebellum. The dura over the cerebellum is carefully removed.

To study the acute effect of tapentadol or its salt on *locus coeruleus* neurons *in vivo,* dose-effect curves can be performed. It is injected at 2 min intervals, in increasing doses, until maximal effect is reached. Furthermore, the possible role of the vehicle used to dissolve tapentadol or its salt on the activity of *locus coeruleus* neurons is studied. For this purpose, the same protocol used to study the effect of tapentadol or its salt is followed.

### Electrophysiological recording of locus coeruleus neurons in vivo:

The recording electrode is an Omegadot single-barrel glass micropipette filled with a 2% solution of Pontamine Sky Blue in 0.5% sodium acetate and broken back to a tip diameter of 1 - 2.5 µm. The extracellular signal from the electrode is amplified, discriminated and monitored on an oscilloscope and with an audio monitor too. Discriminated spikes are fed into a PC and processed using computer software (CED micro 1401 interface and Spike2 software, Cambridge Electronic Design, U.K.). *Locus coeruleus* neurons are encountered 5.5 - 6.0 mm below the dural surface, just ventral to a zone of relative silence (corresponding to the IV^{th} ventricle), and medial to neurons of the mesencephalic nucleus of the V^{th} cranial nerve (which could be activated by depression of the mandible). *Locus coeruleus* neurons are identified by standard criteria that include: long duration action potential (> 2 ms); spontaneous firing at a regular rhythm; a slow firing rate; and characteristic spikes with a long-lasting positive-negative waveform. The basal firing rate is recorded at least 2 min prior to any drug administration. Only one noradrenergic *locus coeruleus* cell is pharmacologically studied in each animal.

### Data and statistical analysis:

Frequency rate is expressed in hertz (Hz) and the changes in firing rate as percentages of the baseline firing rate (defined as 0%). Dose-concentration-effect curves are analyzed for the best non-linear fit to a logistic three-parameter equation: E = Eₘₐₓ [A]ⁿ/(ED₅₀ⁿ + [A]ⁿ), where [A] is the i.v. dose of tapentadol or its salt and E is the effect on the firing rate induced by A; Eₘₐₓ is the maximal percentage change at "infinite" dose (100 %); ED₅₀ is the effective dose for eliciting 50 % of Eₘₐₓ; n is the slope factor of the dose-response curve. Experimental data is analyzed by using the computer program Graphd Prism (v. 3.0; GraphPad Software, Inc.). Statistical significance is assessed by means of a one-way repeated measures analysis of variance (ANOVA) to study the effect of compounds on spontaneous firing rate. To analyze the firing rate before and after drug administration, unpaired Student's t-tests is used. The level of significance is considered as p < 0.05. Data is reported as mean ± S.E.M.

### Model 2:

Musculoskeletal pain is the hallmark of fibromyalgia. A model has been developed by K. A. Sluka which has greater face validity to pain of musculoskeletal origin in humans.

This model is characterized by robust muscle (primary) and cutaneous (secondary) hyperalgesia induced by repeated intramuscular acid injections.

### Method:

### Experimental procedures:

The experiments can be performed on adult Sprague-Dawley rats, for example, housed in transparent plastic cages with free access to food and water, in a 12 h light-dark cycle. All the experimental procedures can be approved, for example, by the Animal Care and Use Committee at the University of Iowa.

### Non-Inflammatory Muscle Pain:

All rats are anesthetized with 2%-3% isoflurane. The left gastrocnemius muscle is injected with 100 µL of pH 4.0 saline, 5 days apart. 24 hours after the second injection of acidic saline mechanical withdrawal thresholds of the paw and muscle are measured.

### Behavior test: mechanical withdrawal threshold of the muscle:

Rats are acclimated to the room for 20 minutes. Rats are acclimated for 2 days, 2 times per day for 5 minutes to a gardener's glove prior to testing. To test for muscle withdrawal thresholds the rat is placed in a gardener's glove and the gastrocnemius is squeezed with a tweezer apparatus until a withdrawal of the hindlimb. This is repeated three times and averaged to obtain a muscle withdrawal threshold.

### Behavior test: Mechanical withdrawal threshold of the paw:

Rats are acclimated to the room for 20 minutes and to the testing transparent plastic cages on elevated wire mesh floor for 15 minutes for 2 days prior to testing. To test for mechanical withdrawal thresholds of the paw calibrated von Frey filaments with bending forces ranging from 1 to 210 mN are applied to the both the ipsilateral and contralateral paws. Each filament is applied for approximately 1 s with enough force to bend the filaments. Each filament is applied twice and a positive response is one withdrawal. Once a positive response is found, the filament above and below the filament that caused a positive response is tested. Confirmation of withdrawal threshold is established if there is a positive withdrawal from the filament above and no withdrawal from the filament below. The lowest withdrawal force that produces a withdrawal is recorded as the threshold. A decrease in mechanical withdrawal threshold of the paw is interpreted as cutaneous hyperalgesia of the paw.

## Claims

1. Tapentadol for use in the treatment of fibromyalgia or chronic fatigue syndrome.

2. Tapentadol according to claim 1, wherein the fibromyalgia is selected from fibromyositis, fibrositis, myofibrositis, diffuse myofascial pain syndrome, primary fibromyalgia, secondary fibromyalgia, fibromyalgia-fibromyositis syndrome, fibromyositis-fibromyalgia syndrome, and muscular rheumatism.

3. Tapentadol according to claim 1 or 2, wherein it is present in a medicament.

4. Tapentadol according to claim 3, wherein the medicament is solid.

5. Tapentadol according to claim 3 or 4, wherein the medicament is formulated for oral administration.

6. Tapentadol according to any one of claims 3-5, wherein the medicament is a tablet.

7. Tapentadol according to any one of claims 3-6, wherein the medicament is formulated for administration twice daily (*bid*).

8. Tapentadol according to any one of claims 3-7, wherein the medicament contains tapentadol in an amount of 10 to 300 mg.
